(19) Europäisches Patentamt
European Patent Office
Office européen des brevets

(11) EP 0 423 230 B1

(12) EUROPEAN PATENT SPECIFICATION

(45) Date of publication and mention of the grant of the patent: **24.01.1996 Bulletin 1996/04**

(21) Application number: **89908613.6**

(22) Date of filing: **29.06.1989**

(51) Int Cl.[6]: **C07C 59/58**, C07C 59/305, C07C 69/60

(86) International application number: **PCT/US89/02896**

(87) International publication number: **WO 90/00538 (25.01.1990 Gazette 1990/03)**

(54) **ANIONIC SURFACTANT ADDITION PRODUCTS OF MALEIC OR FUMARIC ACID AND A POLY(ETHOXYLATED) ALCOHOL**

ANIONISCHE OBERFLÄCHENAKTIVE ADDITIONSPRODUKTE VON MALEIN ODER FUMARSÄURE UND EINEM POLY(ETHOXYLIERTEN) ALKOHOL

TENSIO-ACTIFS ANIONIQUES RESULTANT D'UNE REACTION D'ADDITION ENTRE ACIDE MALEIQUE OU FUMARIQUE ET UN ALCOOL POLY(ETHOXYLE)

(84) Designated Contracting States: **DE FR GB**

(30) Priority: **05.07.1988 US 215169**

(43) Date of publication of application: **24.04.1991 Bulletin 1991/17**

(73) Proprietor: **OLIN CORPORATION**
**Stamford, Connecticut 06904 (US)**

(72) Inventors:
- **HEMLING, Thomas, C.**
  **Meriden, CT 06450 (US)**
- **WEAVER, Otha, G., Jr.**
  **Prospect, CT 06712 (US)**

(74) Representative: **Baker, Colin John et al**
**London EC1N 2JT (GB)**

(56) References cited:
**US-A- 2 264 103**
**US-A- 3 340 309**
**US-A- 3 629 121**
**US-A- 3 954 858**
**US-A- 3 976 586**
**US-A- 4 533 485**
**US-A- 4 533 486**
**US-A- 4 548 729**
**US-A- 4 624 803**

Remarks:
The file contains technical information submitted after the application was filed and not included in this specification

EP 0 423 230 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

This invention relates generally to anionic surfactants and, more specifically, to such surfactants which exhibit excellent sequestering efficacy for metal ions. These surfactants are prepared by the additions reaction of maleic or fumaric acid with a poly(ethoxylated) alcohol.

Anionic surfactant addition products of maleic or fumaric acid and certain types of poly(oxyalkylated) alcohols are known in the art. By way of illustration, U.S. Patent No. 4,533,485 discloses such surfactants wherein the oxyalkylated moiety contains at least two different alkoxy groups, such as blocks of ethylene oxide and propylene oxide groups. Column 7, lines 35-48, of this patent provides an explanation for the requirement for both of these different groups by stating that the ethylene oxide block is susceptible to degradation in caustic and other alkaline solutions, thereby causing incompatibility in various industrial and institutional applications.

The surfactants disclosed in the above '485 patent exhibit excellent physical properties, and some have attained wide commercial acceptance. However, these surfactants are structurally not as simple as might be desired due to the requirement for two different kinds of alkoxy groups. In addition, the fabrication time for certain of these ethylene oxide and propylene oxide block-containing materials can be longer than might be desired.

In the past, phosphates have been commonly used as sequesterants for metal ions, typically in household detergents. However, due to recent federal and state legislation banning the use of phosphates in such applications, new methods of sequestering metal ions, such as calcium, magnesium, copper or iron ions, would be highly desired by the household and institutional cleaninq products communities. In addition, new surfactants which are easier and simpler to fabricate and which exhibit a combination of caustic solubility and stability in alkali media, together with metal ion sequestering efficacy, would be highly desired by the surfactant industry. Heretofore, such new surfactants had not been discovered to the knowledge of the present inventors.

In one aspect, the present invention relates to a surfactant composition obtainable by a process comprising :

a) forming a carboxylic acid addition product by reacting, in the presence of a peroxy-type free radical initiator, an ethylenically unsaturated dicarboxylic acid or derivative selected from maleic acid, maleic anhydride, fumaric acid, and mixtures thereof, with at least one poly(ethoxylated) alcohol having the empirical structural formula:

$$R_1\text{-0-}(CH_2\text{-}CH_2\text{-}0)_x\text{-}R_2$$

wherein $R_1$ is linear $C_6$-$C_{18}$ alkyl (preferably a linear $C_9$-$C_{16}$ alkyl); $R_2$ is hydrogen; and <u>x</u> is a number having a value of between about 1 and about 25; the weight ratio of said poly(ethoxylated) alcohol to said dicarboxylic acid being between about 95:5 and about 40:60 (preferably between about 90:10 and about 50:50), the number of acid groups per ethoxy group in the alcohol in the reaction mixture being between about 0.4 and about 0.6; and

b) neutralizing said addition product with a sufficient amount of a neutralizing agent to convert at least a major portion of said carboxylic acid group to salt group,

said surfactant composition having an aqueous surface tension of less than 45 (preferably between 25 and 35) dynes/cm and a calcium sequestering efficacy of at least 20 milligrams of calcium per gram of surfactant composition.

In another aspect the present invention relates to a composition having an empirical structural formula of:

$$R_1\text{-O-}(\underset{Y}{CH}\text{-}\underset{Z}{CH}\text{-O})_x\text{-}R_2$$

wherein $R_1$ is linear $C_6$-$C_{18}$ alkyl (preferably a linear $C_9$-$C_{16}$ alkyl); $R_2$ is hydrogen; <u>x</u> is a number having a value of between about 1 and about 25; and wherein Y and Z are individually selected from hydrogen and a radical derived from maleic acid, maleic anhydride, fumaric acid, and mixtures thereof, (such as a succinic acid radical) with the proviso that at least one succinic acid radical be present per molecule of said composition. Preferably, the radical is present on average in an amount of between about 10 weight percent and about 50 weight percent based upon the total weight of said composition.

These and other aspects of the present invention will become apparent upon reading the following detailed description of the invention.

In accordance with the present invention, it has now been surprisingly found that the addition products, maleic acid, fumaric acid, or mixtures thereof, with a select poly(ethoxylated) alcohol provides a surfactant having excellent calcium sequestering efficacy. Moreover, the surfactant is physically and chemically stable in aqueous alkali media. Moreover, this surfactant is produced by a straight-forward reaction that is relatively fast as compared to certain of the analogous reactions using poly(ethoxylated-propoxylated) alcohols of the prior art.

While the present invention is not to be limited, this free radical initiated addition reaction is believed to occur by a three-step mechanism, which is illustrated by the following Equations (I) through (X) wherein the poly(ethoxylated) alcohol employed is represented by A one of the selected acids is represented by B; and the peroxy-type free radical initiator is represented by ROOR:

Initiation:

$$ROOR \rightarrow 2RO. \quad (I)$$

Propogation:

$$A + RO. \rightarrow A. + ROH \quad (II)$$

$$A. + B \rightarrow A\text{-}B. \quad (III)$$

$$A\text{-}B. + A \rightarrow A\text{-}B + A. \quad (IV)$$

$$A\text{-}B. + ROH \rightarrow A\text{-}B + RO. \quad (V)$$

Termination:

$$A. + A. \rightarrow A\text{-}A \quad (VI)$$

$$AB. + A. \rightarrow A\text{-}B\text{-}A \quad (VII)$$

$$AB. + AB. \rightarrow ABBA \quad (VIII)$$

Maleic acid, fumaric acid and mixtures thereof [maleic acid being [cis--HOOCCH--CHCOOH] and fumaric acid being (trans--HOOCCH--CHCOOH)], are suitable ethylenically unsaturated dicarboxylic acids since they do not readily homopolymerize under the reaction conditions of the present invention. Free radical addition reactions with them are completed by removal of a hydrogen from another poly(ethoxylated) alcohol [see Equation (IV) above] or from another hydrogen atom source. Furthermore, it has been found that these carboxylic acids [when reacted with poly(ethoxylated) alcohols according to the present invention] are particularly suitable formaking surfactants having excellent sequestering ability and additionally alkaline stability and solubility.

In accordance with the present invention, the dicarboxylic acid reacts with the poly(ethoxylated) alcohol to form the addition product by replacing a hydrogen on at least one of the carbons on at least one of the ethylene oxide substituents per molecule. In theory, each of such carbon atoms could act as a site for attachment of a carboxylic acid group. In practice, however, steric effects would normally be expected to limit the number of carboxylic acid groups that add to each molecule of alcohol to a small fraction of the theoretical number of bonding sites. Generally, the number of acid groups per ethoxy group on the alcohol in the reaction mixture will be between about 0.4 and about 0.6.

Poly(ethoxylated) alcohols may be made by methods analogous to those described in U.S. Patent No. 3,340,309 at column 3. The disclosure of this patent is incorporated herein by reference in its entirety. Generally, poly(ethoxylated) alcohols may be made by condensing an aliphatic alcohol, or mixture of alcohols, of the desired average chain length, with ethylene oxide. The methods used for condensing may be any of the well-known methods described in the art. Preferably, the reaction to make the intermediate occurs at elevated temperatures in the range of about 140°C to about 200°C (more preferably from about 160°C to 180°C). It is also preferred to carry out such reactions in the presence of an effective amount (e.g. about 0.005 percent to 1 percent by weight of the alcohol weight) of a suitable alkaline catalyst (s) such as salts or hydroxides of the alkali metals or alkaline earth metals. The preferred catalyst is KOH. Various methods for making poly(ethoxylated) alcohols are known and many of these intermediates are commercially available.

The $R_1$ moiety on the poly(ethoxylated) alcohols is suitably a straight-chain alkyl having an average number of carbons as specified above. Typically, $R_1$ will comprise a hydrophobic mixture of hydrocarbon radicals such as a $C_9$-$C_{11}$ mixture, a $C_{12}$-$C_{15}$ mixture or a $C_{16}$-$C_{18}$ mixture whereas $R_2$ is a hydrogen moiety.

In fabricating the surfactant composition of the present invention, it should be noted that peroxy-type free radical initiators are preferred. Other types of initiators are less preferred for this reaction. Typical peroxy-type free radical initiators include hydrogen peroxide and organo peroxides and hydroperoxides such as dibenzoyl peroxide, acetyl peroxide, benzoyl hydroperoxide, t-butyl hydroperoxide, di-t-butyl peroxide, lauroyl peroxide, butyryl peroxide, diisopropylbenzene hydroperoxide, cumene hydroperoxide, paramenthane hydroperoxide, diacetyl peroxide, dialphacumyl peroxide, dipropyl peroxide, diisopropyl peroxide, isopropyl-t-butyl peroxide, butyl-t-butyl peroxide, dilauryl peroxide, difuroyl peroxide, ditriphenylmethyl peroxide, bis(p-methoxy-benzoyl) peroxide, p-monomethoxybenzoyl peroxide, rubrene peroxide, ascaridol, t-butylperoxybenzoate, diethyl peroxyterephthalate, propyl hydroperoxide, isopropyl hydroperoxide, n-butyl hydroperoxide, t-butyl hydroperoxide, cyclohexyl hydroperoxide, trans-Decalin hydroperoxide, alpha-methylbenzyl hydroperoxide, alpha-methyl-alpha-ethyl benzyl hydroperoxide, Tetralin hydroperoxide, triphenylmethyl hydroperoxide, diphenylmethyl hydroperoxide, 2,5-di-methyl-2,5-bis(2-ethyl hexanoyl peroxy)hexane, 1,1-bis(t-butyl-peroxy) cyclohexane and t-butyl perbenzoate.

As stated above, the weight ratio of the total poly(oxyalkylated) alcohol(s) to the unsaturated dicarboxylic acid employed in the addition reaction is generally between about 95:5 and about 40:60 (preferably between about 90:10 and about 50:50). When less than about 5 parts by weight of the acid is used per about 95 parts of the alcohol, the resulting composition is not expected to provide sequestering efficacy. When more than about 50 parts by weight of the acid is employed per about 50 parts of the alcohol, a significant portion of the acid will frequently not react onto the alcohol due to steric hindrance of reactive sites depending upon the specific structure of the alcohol.

Besides the specified reactants, peroxy-type initiators and weight ratios mentioned above, the other reaction conditions for the addition reaction are not critical to the present invention and the present process should not be limited to any particular conditions. It is preferred to carry out the addition reaction at a temperature of between about 25°C and about 150°C. More preferably, the reaction temperature may be in the range of between about 80°C and about 150°C. The reaction temperature should be high enough to activate the peroxy-type free radical initiator for this reaction. In some cases, it may be desirable to add a free radical accelerator such as ferrocene or a Redox catalyst to speed up the reaction. The reaction time will depend mainly upon the reation temperature used, the specific alcohol reactant employed, and the molar ratio of acid to alcohol desired to be reacted. Suitable reaction times will range from 1/2 hour up to about 25 hours; preferably between 1/2 hour and 5 hours. The reaction maybe monitored by following the disappearance of the acid or anhydride in the reaction mixture using conventional analysis techniques.

Generally, this reaction is carried out without a solvent. However, in some cases, it may be desirable to employ an organic solvent. For example, if a very viscous poly(oxyalkylated) alcohol is employed, it may be desirable to thin the reaction mixture by using an organic solvent or aqueous emulsion polymerization to facilitate the reaction.

Reaction pressure can be selected as desired. Typically, super- or sub-atmospheric reaction pressure is not necessary for the present reaction. Atmospheric pressure is preferred in order to avoid the expense of special reaction vessels. However, elevated pressures can be used, if desired, and these superatmospheric pressures of up to 10 atmospheres or higher are suitably utilized to drive the reaction to completion and thereby reduce the reaction time.

The free radical initiated reaction of this invention may be conducted under conditions known to be suitable for free radical polymerizations. The reaction is advantageously carried out by mixing the reactants, initiator(s), and optionally with a free radical accelerator(s) and solvent, at temperatures from about 25°C, to about 150°C with an inert atmosphere (e.g., under a nitrogen blanket) until the reaction is complete. The in itiator(s) and optional catalyst(s) and solvent may be added at the beginning of the reaction or may be added portionwise at intervals during the course of reaction. Likewise, the unsaturated acid reactant(s) and the poly(oxyalkylated) alcohol(s) reactants may be brought together at the beginning of the reaction or may be combined in increments as the reaction proceeds.

The adducts produced by this reaction are generally water-insoluble, but they may be converted into water-dispersible or water-soluble form by reaction with a conventional neutalization agent (e.g. an inorganic or organic base) which converts some or all of the carboxylic acids groups into ionic groups.

Thus, the formed addition product is neutralized in accordance with this invention in order to convert at least a major portion (i,e., at least 50 percent) of the carboxylic acid groups on the addition product into ionic groups.

Any conventional neutralizing agent may be employed. Preferred agents include water soluble primary, secondary, or tertiary amines (e.g. triethanolamine), alkali metal hydroxides and mixtures thereof. The most preferred neutralization agents are sodium hydroxide and potassium hydroxide.

The amount of neutralization agent added is preferably sufficient to convert substantially all (i.e., above about 95 percent) of the carboxylic acid groups in the addition product to salt groups (e.g., $\text{--COO}^-\text{Na}^+$). The presence of these salt groups allow the composition to be alkali- and water-soluble. It should be noted that the neutralization agent may also be a alkali-containing processing bath or the like in which the surfactant is to be used. In this latter case, it may be desirable to merely add the unneutralized (or free-acid) adduct of the present invention and allow the neutralization to take place in-situ.

The advantageous properties of the surfactant compositions of the present invention make them suitable for use in dishwashing detergent formulations, laundry detergents, as wetting, washing and dispersing agents in the textile, leather, paper, paint, pharmaceutical and cosmetic industries, as well as for household applications. The anionic surfactants of the present invention are particularly useful as surface active agents or emulsifiers in aqueous mixtures (e.g., solutions, suspensions and the like).

The following examples 1A, and 1E, and Tables, are intended to illustrate, but in no way limit the scope of, the present invention. Examples 1B, 1C and 1D are provided for reference purposes only.

EXAMPLE 1

A. Preparation of a Surfactant Composition (Composition No. 3 in Table I Below) of the Present Invention Having Caustic Solubility

Into a 100 ml, 3-neck, round bottom flask equipped with a thermometer, stirring bar, nitrogen inlet with bubbler, and a water condenser was placed NEODOL(R) 91-8* (29.5 g; 0.058 moles; a product of the Shell Oil Company). The contents of the flask were then heated to 100°C under vacuum (5-10 mm) for 10 minutes to remove any water present. The flask was then cooled to 70°C and fumaric acid (25.9 g; 0.23 moles) was added followed by 1.5 g di-t-butyl peroxide. The reaction mixture was heated at 125° to 130°C for six hours. Then another 1.7 g di-t-butyl peroxide was added and the mixture heated at 128° to 132°C for 10 hours. The clear yellow solution was cooled to 100°C and diluted with 40.2 g of

* NEODOL(R) 91-8 is a $C_9$-$C_{11}$ linear alcohol ethoxylated with 8 moles of ethylene oxide, manufactured by the Shell Oil Company.

water and then neutralized (pH 9) with 35.5 g of 50 percent NaOH to provide a solution containing 50 weight percent active product. A 0.5 weight percent solution of this product was soluble in a solution of 20 percent sodium hydroxide.

B. Preparation of a Reference Surfactant Composition (Reference Composition No. 8 in Table I Below)

Using the same equipment as in EXAMPLE 1, ethyl capped BRIJ(R) 35** (10 g; 0.0081 moles; a product of ICI Australia Limited), fumaric acid (5.7 g; 0.049 moles), and 0.75 g of di-t-butyl peroxide were mixed together and heated under nitrogen at 138° to 144°C for two hours; The solution was then cooled slightly (80°C) and 8.55 g of 40 percent NaOH was added dropwise. After addition was completed, 12.4 g of water was added to afford a solution containing 50 percent active material.

C. Preparation of a Second Reference Surfactant Composition (Reference Composition 5 in Table I Below)

Using the same equipment as in EXAMPLE 1 (A) above, 5.07 g (0.0084 moles) of AVANEL(R) S-70*** (100 percent active material; a product of PPG Industries Inc.), 2.94 g (0.25 moles) of fumaric acid, and 0.5 g of di-t-butyl peroxide were added together and heated at 138° to 143°C for eight hours. The viscous product was cooled to approximately 100°C, and the solution neutralized with 4.04 g of 50 percent NaOH followed by 6.3 g of $H_2O$ to give a 50 percent solids solution. A 1.0 weight percent solution of this product was soluble in 30 percent sodium hydroxide solution.

D. Procedure for Making a Reference End-Capped Surfactant

Into a 500 ml three-neck flask equipped with stir bar, $N_2$ bubbler, and condenser was placed 99.6 g of NEODOL(R) 25-12 (a product of the Shell Oil Company) and 225 g of toluene. To this solution was added 3.11 g sodium metal and the mixture heated at 105°C overnight.

Into a second flask was placed 50 ml toluene and 7.0 ml (excess) of EtBr. To this solution was added dropwise over approximately 40 minutes (at 25°C) 200 ml of the sodium alkoxide of NEODOL(R) 25-12. A precipitate started to form almost immediately. The mixture, after addition, was heated at 50°C for 15 hours. The salt was removed by centrifuge and the toluene removed under vacuum to afford an ethyl-capped 25-12. The cloud paint of a 1 percent solution was 67°C compared to 97°C for NEODOL(R) 25-12.

The surfactant properties and sequestering data for several surfactants of this invention prepared by procedures analogous to those described above are described below in TABLE I.

** An ethyl-capped modification of BRIJ 35 which is $C_{12}H_{25}(OCH_2CH_2)_{23}OH$ manufactured by ICI America Limited.
*** An alkyl polyethyoxylated sulfonate (M.W. 600), manufactured by PPG Industries Inc.

**TABLE I**

| Surfactant Composition No. | R1 | R2 | Average # of EO Groups Per Molecule | Average # of Fumaric Acid Groups Per Molecule | NaOH Solubility Wt.% | Draves* Wetting Time (0.5/0.10%) | Ca Sequestering mg Ca/gm Surfactant | Surface Tension dynes/cm Test (0.5 %) |
|---|---|---|---|---|---|---|---|---|
| 1 | $C_{13}$ | H | 6 | 3 | 25 | 7/38 secs. | 31.5 | 28.5 |
| 2 | $C_{13}$ | H | 9 | 4 | 20 | 7/42 | 20.2 | 26 |
| 3 | $C_{9-11}$ | H | 8 | 4 | 20 | 7/>300 | 25.0 | 29 |
| 4 | $C_{12-15}$ | H | 12 | 6 | 25 | 31/305 | 34.2 | 29.5 |
| 5 | $C_{12}$ | $C_2H_4SO_3$ | 7 | 3 | 30 | 41/210 | 15.7 | 27 |
| 6 | $C_{13-15}$ | H | 12 | 6 | 25 | 83/>300 | 14.4 | 33.5 |
| 7 | $C_{18}$ | H | 20 | 9 | 25 | >300/300 | 24.0 | 42 |
| 8 | $C_{12}$ | Et | 23 | 6 | - | - | 16.7 | - |

* "Draves Wetting Time" denotes the time required to wet a 5 g cotton skein in an aqueous solution of surfactant following AATCC Method 17-1952. This property is important to textile processing utility.

Surfactant Composition No's 5,6 and 8 above are shown for reference purposes only.

E. Testing of Surfactant Composition No. 4 From TABLE I for Calcium Sequestering Ability

The sequestering ability of Surfactant Composition 4 from TABLE I is compared to that of STPP or sodium citrate dihydrate in solutions with various amounts of added base is shown in TABLE II below. The procedure used for determining calcium sequestering (Modified Hampshire Method) was as follows:

One ml of 2 percent sodium oxalate is added to 10 ml of a 1 percent aqueous solution of sequestering agent. Solution

is titrated with a calcium acetate solution to identify the calcium sequestering ability of each sequestering agent. End point is determined by a change in turbidity.

TABLE II

| Calcium Sequestering Efficacy of Surfactant Composition 4 (See TABLE I) at Various Levels of Added Base | | | |
|---|---|---|---|
| Number of ml of 6% NaOH Added | STPP | Calcium Sequestered (mg Ca/gm Sequestering Agent) | |
| | | Sodium Citrate (dihydrate) | Example (Surfactant Composition 4) |
| 0 | 94.1 | 39.3 | 6.1 |
| 1 | 96.9 | 37.4 | 32.1 |
| 2 | 38.5 | 50.1 | 35.6 |
| 5 | 40.5 | 92.7 | 67.0 |

TABLE II shows differences in calcium sequestering efficacy at various levels of added base for the tested sequesterants. Surfactant Composition 4 (from TABLE I) has a calcium sequestering ability similar to that of sodium tripolyphoaphate (STPP) and sodium citrate. The sequestering ability of STPP decreased as the amount of added base was increased, while the sequestering ability for sodium citrate and for Surfactant Composition 4 increased as the amount with added sodium hydroxide was increased.

Surfactant Composition 4 was also compared against sodium citrate dihydrate for calcium sequestering ability in a consumer laundry liquid. The results are presented in TABLE III which follows:

TABLE III

| $Ca^{+2}$ Sequestering by Consumer Liquid Laundry | | |
|---|---|---|
| Ingredient | Formulation A Wt. % | Formulation B Wt. % |
| DDBSA--Na | 17.5 | 15 |
| Neodol 25-7 | 6.0 | 5 |
| Sodium Xylene Sulfonated, 40% | 11.5 | -- |
| Triethanolamine, 85% | 2.0 | 2 |
| Sodium Citrate--Dihydrate | 10.0 | -- |
| Surfactant Composition 4 (see TABLE I) | -- | 8.5 |
| Water | 53.0 | 69.5 |
| $Ca^{+2}$ Sequestering (mg/gm Formulation) | 10.2 | 9.4 |
| Weight % Surfactant | 23.5% | 28.5% |

TABLE III shows that the calcium sequestering of Surfactant Composition 4 when tested by itself (TABLE II) carries over into a fully formulated product. The sodium citrate liquid laundry formulation (Formulation A in TABLE III) sequestered 10.2 mg calcium per gram of the formulation, while Formulation B which contains Surfactant Composition 4 (see TABLE I) sequestered 9.4 mg/gram of the formulation. An added benefit provided by Surfactant Composition 4 is that the hydrotrope sodium xylene sulfonate is not needed to hold the composition together.

## Claims

**1.** A surfactant composition obtainable by a process characterized by:

a) forming a carboxylic acid addition product by reacting, in the presence of a

peroxy-type free radical initiator, an ethylenically unsaturated dicarboxylic acid or derivative selected from maleic acid, maleic anhydride, fumaric acid, and mixtures thereof, with at least one poly(ethoxylated) alcohol having the empirical structural formula:

$$R_1\text{-0-}(CH_2\text{-}CH_2\text{-0})_x\text{-}R_2$$

wherein $R_1$ is a linear $C_6$-$C_{18}$ alkyl; $R_2$ is hydrogen; and $\underline{x}$ is a number having a value of between about 1 and about 25; the weight ratio of said poly(ethoxylated) alcohol to said dicarboxylic acid being between

about 95:5 and about 40:60, the number of acid groups per ethoxy group on the alcohol in the reaction mixture being between about 0.4 and about 0.6; and

b) neutralizing said addition product with a sufficient amount of a neutralizing agent to convert at least a major portion of said carboxylic acid group to salt group, said surfactant composition having an aqueous surface tension of less than 45 dynes/cm, and a calcium sequestering efficacy of at least 20 milligrams of calcium per gram of surfactant composition.

**2.** A surfactant composition according to claim 1, wherein said $R_1$ is a linear $C_9$-$C_{16}$ alkyl.

**3.** A surfactant composition according to claim 1 or claim 2, wherein the weight ratio of said poly(ethoxylated) alcohol to said dicarboxylic acid is between about 90:10 and about 50:50.

**4.** A composition obtainable by step (a) in claim 1 having an empirical structural formula of:

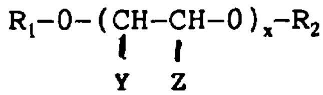

wherein $R_1$ is a linear $C_6$-$C_{18}$ alkyl, $R_2$ is hydrogen; $\underline{x}$ is a number having a value of between about 1 and about 25; and wherein Y and Z are individually selected from hydrogen and a radical derived from maleic acid, maleic anhydride, fumaric acid, and mixtures thereof, with the proviso that at least one of said radicals be present per molecule of said composition.

**5.** A composition according to claim 4 wherein said $R_1$ is a linear $C_9$-$C_{16}$ alkyl.

**6.** A composition according to claim 4 or claim 5, wherein said radical is present on average in an amount of between about 10 weight percent and about 50 weight percent based upon the total weight of said composition.

**7.** A composition according to any one of claims 4 to 6 which has a calcium sequestering efficacy of at least about 20 milligrams of calcium per gram of composition after neutralization according to step (b) of claim 1.

**Patentansprüche**

**1.** Oberflächenaktive Zusammensetzung erhältlich in einem Verfahren, **gekennzeichnet durch**

a) Bildung eines Carbonsäureadditionsproduktes durch Reaktion, in Anwesenheit eines peroxidischen Starters mittels freier Radikale, von einer ethylenisch ungesättigten Dicarbonsäure oder eines Derivats derselben, ausgewählt aus Maleinsäure, Maleinsäureanhydrid, Fumarsäure und Mischungen derselben, mit wenigstens einem poly(ethoxylierten) Alkohol mit der empirischen Strukturformel:

$$R_1\text{-}0\text{-}(CH_2\text{-}CH_2\text{-}0)_x\text{-}R_Z,$$

wobei $R_1$ eine lineare $C_6$-$C_{18}$ Alkylgruppe ist; $R_2$ Wasserstoff ist und $\underline{x}$ eine Zahl mit einem Wert zwischen ungefähr 1 und ungefähr 25 ist; das Gewichtsverhältnis des poly(ethoxylierten) Alkohols zu dem der Dicarbonsäure zwischen ungefähre 95:5 und ungefähr 40:60 ist, die Zahl der Säuregruppen je Ethoxygruppe des Alkohols in der Reaktionsmischung zwischen ungefähr 0,4 und ungefähr 0,6 beträgt und

b) Neutralisation des Additionsproduktes mit einem Neutralisationsmittel, in einer Menge, die ausreicht, zumindest den Hauptanteil der Carbonsäuregruppen in Salze der Carbonsäuren zu überführen, wobei die oberflächenaktive Zusammensetzung eine Oberflächenspannung in Wasser von weniger als 45 dyn/cm ($10^{-3}$ N/m) und ein Calciummaskierungsvermögen von mindestens 20 mg Calcium je Gramm der oberflächenaktiven Zusammensetzung aufweist.

**2.** Oberflächenaktive Zusammensetzung nach Anspruch 1, **dadurch gekennzeichnet,** daß $R_1$ eine lineare $C_9$-$C_{16}$ Alkylgruppe ist.

**3.** Oberflächenaktive Zusammensetzung nach Anspruch 1 oder 2, **dadurch gekennzeichnet,** daß das Gewichtsverhältnis des poly(ethoxylierten) Alkohols zu dem der Dicarbonsäure zwischen ungefähr 90:10 und ungefähr 50:50 beträgt.

**4.** Zusammensetzung erhältlich durch Schritt (a) in Anspruch 1 mit einer empirischen Strukturformel:

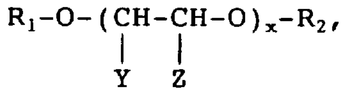

wobei $R_1$ eine lineare $C_6$-$C_{18}$ Alkylgruppe ist; $R_2$ ist Wasserstoff; x ist eine Zahl zwischen ungefähr 1 und ungefähr 25; und wobei Y und Z unabhängig voneinander ausgewählt sind aus der Gruppe bestehend aus Wasserstoff und einem Rest abgeleitet von Maleinsäure, Maleinsäureanhydrid, Fumarsäure und Mischungen derselben unter dem Vorbehalt, daß mindestens einer der genannten Reste je Molekül der Zusammensetzung vorliegt.

**5.** Zusammensetzung nach Anspruch 4, **dadurch gekennzeichnet,** daß $R_1$ eine lineare $C_9$-$C_{16}$ Alkylgruppe ist.

**6.** Zusammensetzung nach Anspruch 4 oder 5, **dadurch gekennzeichnet,** daß der Rest im Mittel in einer Menge zwischen ungefähr 10 Gew.-% und ungefähr 50 Gew.-% des Gesamtgewichtes der Zusammensetzung vorliegt.

**7.** Zusammensetzung nach einem der Ansprüche 4 bis 6, **dadurch gekennzeichnet,** daß das Calciummaskierungsvermögen wenigstens ungefähr 20 mg Calcium je Gramm der Zusammensetzung nach einer Neutralisierung gemäß Schritt (b) von Anspruch 1 beträgt.

**Revendications**

**1.** Composition d'agent tensio-actif que l'on peut obtenir par un procédé caractérisé par :

a) le fait de former un produit d'addition d'un acide carboxylique en faisant réagir en présence d'un déclencheur de radicaux libres du type peroxy, un acide dicarboxylique non saturé éthyléniquement ou un dérivé, choisi parmi l'acide maléique, l'anhydride maléique, l'acide fumarique et les mélanges de ceux-ci, avec au moins un alcool poly(éthoxylé) ayant la formule structurelle brute :

$$R_1\text{-O-}(CH_2\text{-}CH_2\text{-O})_x\text{-}R_2$$

dans laquelle $R_1$ est un radical alcoyle linéaire en $C_6$ à $C_{18}$ ; $R_2$ est de l'hydrogène ; et x est un nombre ayant une valeur comprise entre environ 1 et environ 25 ; le rapport en poids de l'alcool poly(éthoxylé) à l'acide dicarboxylique étant compris entre 95:5 environ et environ 40:60, le nombre de groupes acide par groupe éthoxy dans l'alcool, dans le mélange réactionnel étant compris entre environ 0,4 et environ 0,6 ; et
b) le fait de neutraliser le produit d'addition avec une quantité suffisante d'un agent neutralisant pour convertir au moins une fraction prépondérante du groupe d'acide carboxylique en un groupe sel, la composition d'agent tensio-actif ayant une tension superficielle aqueuse de moins de 45 dynes/cm et une efficacité pour complexation du calcium d'au moins 20 milligrammes de calcium par gramme de composition d'agent tensio-actif;

**2.** Composition d'agent tensio-actif selon la revendication 1, caractérisée en ce que $R_1$ est un alcoyle linéaire en $C_9$-$C_{16}$.

**3.** Composition d'agent tensio-actif selon la revendication 1 ou la revendication 2, caractérisée en ce que le rapport en poids de l'alcool poly(éthoxylé) à l'acide dicarboxylique est compris entre environ 90:10 et environ 50:50.

**4.** Composition que l'on peut obtenir par l'étape a) dans la revendication 1, ayant une formule structurelle brute de :

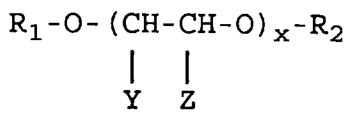

dans laquelle $R_1$ est un alcoyle linéaire en $C_6$ à $C_{18}$ ; $R_2$ est un hydrogène ; x est un nombre ayant une valeur entre environ 1 et environ 25 ; et dans laquelle Y et Z sont individuellement choisis parmi l'hydrogène et un radical dérivé de l'acide maléique, de l'anhydride maléique, de l'acide fumarique, et des mélanges de ceux-ci, avec la restriction qu'au moins un des radicaux est présent par molécule de la composition.

5. Composition selon la revendication 4, caractérisée en ce que $R_1$ est un alcoyle linéaire en $C_9$-$C_{16}$.

6. Composition selon la revendication 4 ou la revendication 5, caractérisée en ce que le radical est présent en moyenne en quantité comprise entre environ 10 % en poids et environ 50 % en poids basée sur le poids total de la composition.

7. Composition selon chacune des revendications 4 à 6, qui a une efficacité de complexation de calcium d'au moins environ 20 milligrammes de calcium par gramme de composition après neutralisation selon l'étape b) de la revendication 1.